(19) Europäisches Patentamt / European Patent Office / Office européen des brevets

(11) **EP 1 548 678 B1**

(12) **EUROPÄISCHE PATENTSCHRIFT**

(45) Veröffentlichungstag und Bekanntmachung des Hinweises auf die Patenterteilung:
**08.04.2009 Patentblatt 2009/15**

(51) Int Cl.:
**G08B 21/06** (2006.01)

(21) Anmeldenummer: **04027032.4**

(22) Anmeldetag: **13.11.2004**

(54) **Verfahren und Computerprogramm zum Erkennen von Unaufmerksamkeiten des Fahrers eines Fahrzeugs**

Method and computer program for the detection of the unawareness of a vehicle's driver

Méthode et logiciel pour la reconnaissance d'un manque d'attention d'un conducteur d'un vehicule

(84) Benannte Vertragsstaaten:
**DE ES FR GB IT SE**

(30) Priorität: **26.11.2003 DE 10355221**

(43) Veröffentlichungstag der Anmeldung:
**29.06.2005 Patentblatt 2005/26**

(73) Patentinhaber: **Daimler AG**
**70327 Stuttgart (DE)**

(72) Erfinder:
• **Bukman, Elisabeth**
**71106 Magstadt (DE)**
• **Galley, Lars Arnim**
**10997 Berlin (DE)**
• **Kuhn, Klaus-Peter, Dr.**
**73655 Plüderhausen (DE)**
• **Neumerkel, Dietmar, Dr.**
**13509 Berlin (DE)**

(74) Vertreter: **JENSEN & SON**
**366-368 Old Street**
**London EC1V 9LT (GB)**

(56) Entgegenhaltungen:
EP-A- 0 147 539    JP-A- 7 093 678
US-A- 5 745 031

**Beschreibung**

Die Erfindung betrifft ein Verfahren und ein Computerprogramm zum Erkennen, wann der Fahrer eines Fahrzeugs, insbesondere eines Kraftfahrzeugs Kfz, während des Betriebs des Fahrzeugs unaufmerksam wird. Darüber hinaus betrifft die Erfindung einen Datenträger mit einem derartigen Computerprogramm.

Aus dem Stand der Technik sind verschiedene Vorschläge bekannt, eine Unaufmerksamkeit eines Kraftfahrzeugführers, insbesondere Einschlaftendenzen des Kraftfahrzeugführers zu erfassen.

Ein entsprechender Vorschlag ist zum Beispiel in der deutschen Offenlegungsschrift DE 198 18 239 A1 offenbart. Die dort offenbarte Vorrichtung zur Einschlafwarnung eines Kraftfahrzeugführers umfasst zum einen eine Fahrzeugumgebungserkennungs-Einrichtung zum Erfassen eines Ist-Fahrstils des Kraftfahrzeugführers. Darüber hinaus umfasst die dort beschriebene Vorrichtung eine Einrichtung zur Erfassung eines Referenzfahrstils, wobei insbesondere detektiert wird, wie viel laterale Eigenbewegungen der Kraftfahrzeugführer gewöhnlich in seinem Fahrstil hat. Schließlich umfasst die offenbarte Vorrichtung eine Vergleichslogik zum Vergleichen des Referenzfahrstils mit dem aktuellen Ist-Fahrstil, um je nach Maßgabe durch das Ergebnis dieses Vergleichs eine Warnung an den Kraftfahrzeugführer auszugeben.

Weiterhin ist aus der US-Patentschrift US 6,061,610 ein Verfahren und eine Vorrichtung zum Bestimmen der Belastung des Fahrers eines Kraftfahrzeugs bekannt. Dieses Verfahren sieht vor, dass der Lenkradwinkel des Kraftfahrzeugs zunächst erfasst wird, um daraus Vorhersagefehler für den Lenkradwinkel bei Steuerung des Kraftfahrzeugs durch den Fahrer zu generieren. Es wird dann eine Verteilung dieser Vorhersagefehler berechnet, um diese Verteilung mit einer anderen Verteilung von Vorhersagefehlern des Lenkradwinkels zu vergleichen, welche nicht das reale Lenkverhalten des Fahrers, sondern ein vorgegebenes Lenkverhalten eines stressfreien beziehungsweise entspannten Fahrers repräsentiert. Das Ergebnis dieses Vergleiches repräsentiert dann die aktuelle Belastung des Fahrers beim Fahren des Kraftfahrzeugs.

Aus der deutschen Offenlegungsschrift DE 25 46 345 ist ein Fahrerwarngerät zur Warnung von Kraftfahrzeugfahrern, bevor sie einschlafen bekannt. Das Gerät erfasst die Lenkbewegung des Fahrers des Kraftfahrzeugs, wobei vorausgesetzt wird, dass bei wachem Fahrer das Lenkrad auch bei einer Geradeausfahrt nicht gänzlich ruhig gehalten wird, sondern dass auch dann ständig wenn auch noch so kleine Lenkbewegungen stattfinden. Wenn das Fahrerwarngerät ein Ausbleiben dieser Lenkbewegungen auch über ein einstellbares Zeitintervall hinaus feststellt, so schließt das Fahrerwarngerät daraus, dass der Fahrer eingeschlafen ist oder zumindest droht einzuschlafen und es warnt dann den Fahrer durch Ausgabe eines Signals.

Dem in der zuletzt genannten Offenlegungsschrift DE 25 46 345 offenbarten Fahrerwarngerät haftet der Nachteil an, dass die Erkennung, wann der Fahrer eines Fahrzeugs unaufmerksam wird, alleine aufgrund der Detektion einer Lenkruhephase erfolgt und deshalb nur recht vage und unzuverlässig möglich ist.

In der JP 07093678 A wird eine Vorrichtung zur Erkennung von Müdigkeit über die Erkennung einer Lenkruhephase mit anschließender Erkennung einer korrigierenden Lenkaktion beschrieben.

In EP 0 147 539 A2 wird ein Verfahren zum Erkennen, wann ein Fahrer eines Fahrzeuges unaufmerksam wird beschrieben. Die Unaufmerksamkeit wird in Abhängigkeit von einer Lenkruhephase und einer anschließenden Lenkaktion bestimmt, wobei die Richtung der Lenkaktion, deren Geschwindigkeit und der Ausschlag der Lenkaktion berücksichtigt werden.

In US 5745031 wird ein System zum Erkennen von Fahrer-Unaufmerksamkeiten beschrieben, bei dem ein Lenkwinkelsensor und eine Kamera zum Aufnehmen der Straßenführung verwendet werden.

Ausgehend von diesem Stand der Technik ist es deshalb die Aufgabe der Erfindung, ein Verfahren und ein entsprechendes Computerprogramm zum Erkennen, wann der Fahrer eines Fahrzeugs unaufmerksam wird, sowie einen Datenträger mit diesem Computerprogramm bereitzustellen, welche ein zuverlässigeres Erkennen einer eventuellen Unaufmerksamkeit des Fahrers ermöglichen.

Diese' Aufgabe wird durch das in Patentanspruch 1 beanspruchte Verfahren gelöst.

Vorteilhafterweise unterscheidet die Erfindung beim Erkennen einer Unaufmerksamkeit des Fahrers zwischen einer Lenkruhephase und einer sich bei Vorliegen eines Zustandes der Unaufmerksamkeit typischerweise daran anschließenden mehr oder weniger hektischen Lenkaktion. Ein Zustand der Unaufmerksamkeit wird also gemäß der Erfindung überhaupt nur dann angenommen, wenn sowohl die Lenkruhephase wie auch die anschließende Lenkaktion in Kombination miteinander erkannt werden; im Umkehrschluss bedeutet dies, dass das alleinige Erkennen einer Lenkruhephase oder einer Lenkaktion nicht ausreichend ist, um auf eine Unaufmerksamkeit des Fahrers zu schließen. Für die Ermittlung des Maßes der Schwere der Unaufmerksamkeit werden die festgestellten Ausprägungen von sowohl der Ruhephase wie auch der Lenkaktion miteinander verknüpft und das Ergebnis dann bewertet.

Damit eine Lenkruhephase erkannt wird, muss der erfasste Lenkradwinkel innerhalb eines Lenkradwinkelintervall liegen. Gemäß der Erfindung ist dieses Lenkradwinkelintervall von der Fahrzeuggeschwindigkeit abhängig. Damit wird berücksichtigt, dass Lenkbewegungen bei hohen Geschwindigkeiten des Fahrzeugs typischerweise geringer sind als bei niedrigen Geschwindigkeiten.

In modernen Fahrzeugen, insbesondere Kraftfahrzeugen, ist ein Sensor zur Erfassung des Lenkradwinkels x normalerweise ohnehin vorhanden. Für die Realisierung des beschriebenen Verfahrens bedarf es deshalb vorteilhafterweise grundsätzlich keines zusätzlichen Sensors.

In der Beschreibung werden zwei verschiedene Ausführungsbeispiele dargestellt, wobei das zweite Ausführungsbeispiel teilweise auf das erste Ausführungsbeispiel Bezug nimmt. Die Patentansprüche 1 bis 4 beziehen sich ausschließlich auf das zweite Ausführungsbeispiel, während die nachgeordneten Ansprüche 5-14 Weiterbildungen des Verfahrens beschreiben, die allgemein von einem Maß für eine Unaufmerksamkeit des Fahrers ausgehen und auch in Kombination mit dem ersten Ausführungsbeispiel anwendbar sind. Erwähnungen des ersten Ausführungsbeispiels sind daher nur zum besseren Verständnis angeführt.

Vorteile des ersten Ausführungsbeispiels

Die Berechnung der Ausprägung der Lenkaktion alleine durch Bildung einer Lenkradwinkelvarianz würde lediglich das Lenkverhalten des Fahrers zu einem Zeitpunkt repräsentieren. Gerade für eine zuverlässige Aussage über das Vorhandensein von Unaufmerksamkeiten ist es jedoch wichtig, auch Änderungen des Lenkverhaltens über der Zeit zu berücksichtigen. Diesem Aspekt wird erfindungsgemäß dadurch Rechnung getragen, dass in die Bildung des Varianz-Verhältnisses zwei Lenkradwinkelvarianzen einfließen, welche jeweils die Lenkaktion des Fahrers zu unterschiedlichen Zeitpunkten repräsentieren, wobei die beiden Zeitpunkte um ein Zeitintervall $\Delta t$ zueinander versetzt liegen.

Vorteilhafterweise kann das erfindungsgemäß berechnete Varianz-Verhältnis bereits als Maß für die Schwere der Unaufmerksamkeit des Fahrers beim Lenken des Fahrzeugs zum Zeitpunkt $t_1$ interpretiert werden; eine Unaufmerksamkeit des Fahrers ist insbesondere dann gegeben, wenn dieses Varianz-Verhältnis einen Wert größer 1 hat.

Vorteile des zweiten Ausführungsbeispiels

Im Unterschied zu dem ersten Ausführungsbeispiel werden bei dem zweiten Ausführungsbeispiel wesentlich weniger Parameter zur Beurteilung der Unaufmerksamkeit des Fahrers herangezogen. Deshalb ist es weniger speicherintensiv. Außerdem ist es aufgrund der Verwendung wesentlich einfacherer Algorithmen leichter beherrschbar und in Echtzeit implementierbar. Insgesamt ist es deshalb für den praktischen Einsatz im Fahrzeug gut geeignet.

So ist es von Vorteil, dass eine Beurteilung der Ausprägung der Lenkruhephase, das heißt ihrer Zeitdauer vorzugsweise lediglich durch Auswerten des Lenkradwinkels und die Ausprägung der Lenkaktion vorzugsweise lediglich durch Erfassen des maximal auftretenden Gradienten des Lenkradwinkels ermittelt wird. Eine Berechnung beziehungsweise Auswertung auf Basis der Varianzfunktion ist deshalb entbehrlich.

Die Verknüpfung der Ausprägung der Lenkruhephase mit der Ausprägung der Lenkaktion zum Ermitteln eines Maßes für die Schwere der Unaufmerksamkeit des Fahrers erfolgt bei dem zweiten Ausführungsbeispiel durch einen mehrdimensionalen Operator. Um unnötigen Rechenaufwand zu sparen, wird diese Verknüpfung jedoch nur dann durchgeführt, wenn sowohl die Lenkruhephase wie auch die erwartete anschließende Lenkaktion jeweils mit einer vorbestimmten Mindestausprägung vorkommen. Sollte die Lenkruhephase oder die Lenkaktion nicht ausreichend stark ausgeprägt sein, so wird erfindungsgemäß angenommen, dass sich der Fahrer nicht in einem Zustand der Unaufmerksamkeit befindet.

Vorteile der Ausgestaltungen des erfindungsgemäßen Verfahrens, die beiden Ausführungsbeispielen gemeinsam sein können.

Vorteilhafterweise lassen sich das Ergebnis der Verknüpfung aus dem ersten oder zweiten Ausführungsbeispiel, das heißt das Varianz-Verhältnis oder das Ergebnis der Operator-Verknüpfung mit Hilfe der Sigmoid-Funktion auf einen Wahrscheinlichkeitswert abbilden. Auf diese Weise ist es möglich, eine Wahrscheinlichkeit zwischen 0 und 100 % dafür anzugeben, dass der Fahrer zum Zeitpunkt $t_1$ beim Lenken des Fahrzeugs unaufmerksam war.

In einer weiteren vorteilhaften Ausgestaltung ermöglicht das beanspruchte Verfahren auf Basis des zuvor ermittelten Wahrscheinlichkeitswertes eine Aussage darüber, mit welcher Wahrscheinlichkeit das Verhalten des Fahrers einer bestimmten, von einer Vielzahl vorgegebener und geeignet ausgewählter Mündigkeitsstufen zuzuordnen ist. Eine derartige Zuordnung erfolgt erfindungsgemäß immer unter Berücksichtigung des aktuell erfassten Lenkradwinkels.

Vorteilhafterweise kann diese Zuordnung zu den vorgegebenen Müdigkeitsstufen dadurch präzisiert werden, dass neben dem Lenkradwinkel als erstem Indikator auch weitere beobachtbare Indikatoren für die Unaufmerksamkeit des Fahrers, wie zum Beispiel dessen Lidschlussverhalten oder dessen Reaktionszeit berücksichtigt werden.

Es ist weiterhin von Vorteil, dass die Einschätzung über die Müdigkeit des Fahrers außerdem dadurch präzisiert werden kann, dass für diese Einschätzung nicht lediglich die aktuell erfassten Werte von insbesondere den genannten Indikatoren, sondern auch die in der jüngeren Vergangenheit vorgenommenen Müdigkeitseinstufungen berücksichtigt werden. Anders ausgedrückt ermöglicht diese Vorgehensweise eine Plausibilitätsprüfung der neuen Einschätzung unter Berücksichtigung der Tatsache, dass die Müdigkeit des Fahrers kein Phänomen ist, welches plötzlich auftritt und wieder verschwindet, sondern welches sich vielmehr nur stetig im Zeitablauf verändert.

Vorteilhafterweise ermöglicht das beanspruchte Verfahren nicht nur, wie bisher beschrieben, einen Rückschluss auf die Müdigkeit des Fahrers als Ursache für die detektierte Unaufmerksamkeit. Vielmehr ermöglicht es auch einen Rückschluss auf andere Ursachen für die detektierte Unaufmerksamkeit, welche zum Beispiel in einem geführten Gespräch mit einem Beifahrer oder der Bedienung einer Vorrichtung, zum Beispiel des Radios oder des Handschuhfachs im Fahrzeug, liegen können.

Um die Zuverlässigkeit einer Aussage über die Aufmerksamkeit beziehungsweise Unaufmerksamkeit des Fahrers des Fahrzeugs zu erhöhen, ist es empfehlenswert, nicht lediglich ein Ergebnis der Verknüpfung nach dem ersten oder zweiten Ausführungsbeispiel auszuwerten, sondern stattdessen diese Aussage auf eine Vielzahl derartiger Verknüpfungsergebnisse zu stützen. Dabei kann diese Mehrzahl sowohl lediglich aus Ergebnissen des ersten oder des zweiten Ausführungsbeispiels, jedoch auch aus einer Mischung von Ergebnissen des ersten und des zweiten Ausführungsbeispiels bestehen. Konkret kann eine Aussage über die Unaufmerksamkeit des Fahrers dadurch zuverlässiger getroffen werden, dass jedes aus einer Verknüpfung resultierende Ergebnis mit einem zugeordneten Gewichtungsfaktor gewichtet wird, um dann aus der vorliegenden Mehrzahl von gewichteten Verknüpfungsergebnissen durch mathematische Mittelwertbildung letzten Endes ein gemitteltes Verknüpfungsergebnis zu gewinnen. Dieses gemittelte Verknüpfungsergebnis repräsentiert dann im Vergleich zu einem nicht gemittelten Verknüpfungsergebnis ein zuverlässigeres Maß für die Schwere der Unaufmerksamkeit des Fahrers beim Lenken des Fahrzeugs zu einem bestimmten Zeitpunkt.

Schließlich ist es von Vorteil, wenn insbesondere der Fahrer des Fahrzeugs über die erkannte Unaufmerksamkeit in Form eines optischen oder akustischen Warnhinweises informiert wird.

Weitere vorteilhafte Ausgestaltungen des Verfahrens sind Gegenstand der abhängigen Ansprüche.

Die oben genannte Aufgabe der Erfindung wird weiterhin durch ein Computerprogramm zur Durchführung des beschriebenen Verfahrens, durch einen Datenträger mit dem Computerprogramm sowie durch ein Steuergerät zum Durchführen des beschriebenen Verfahrens gelöst. Die Vorteile dieser Lösungen entsprechen den oben mit Bezug auf das beschriebene Verfahren genannten Vorteilen.

Vorteilhafterweise braucht dieses Computerprogramm zumindest für einzelne Fahrzeugtypen nur einmal programmiert zu werden und kann dann in allen Fahrzeugen einer entsprechenden Serie implementiert werden.

In der Zeichnung zeigen:

Fig. 1      ein Steuergerät gemäß der Erfindung;
Fig. 2      ein Beispiel für den Verlauf des Lenkwinkels x bei Vorliegen einer Unaufmerksamkeit des Fahrers gemäß der Erfindung;
Fig. 3a     den Ablauf des Verfahrens gemäß dem ersten Ausführungsbeispiel;
Fig. 3b, c  in Verbindung mit Figur 3a den Ablauf des erfindungsgemäßen Verfahrens gemäß dem zweiten Ausführungsbeispiel;
Fig. 4      eine Sigmoid-Funktion;
Fig. 5      ein erstes Ursache-Wirkungs-Diagramm;
Fig. 6      Gauß-Verteilungen für verschiedene Ausprägungsstufen, in welchen ein Wahrscheinlichkeitswert, der die Unaufmerksamkeit des Fahrers repräsentiert, auftreten kann; und
Fig. 7      ein zweites Ursache-Wirkungs-Diagramm.

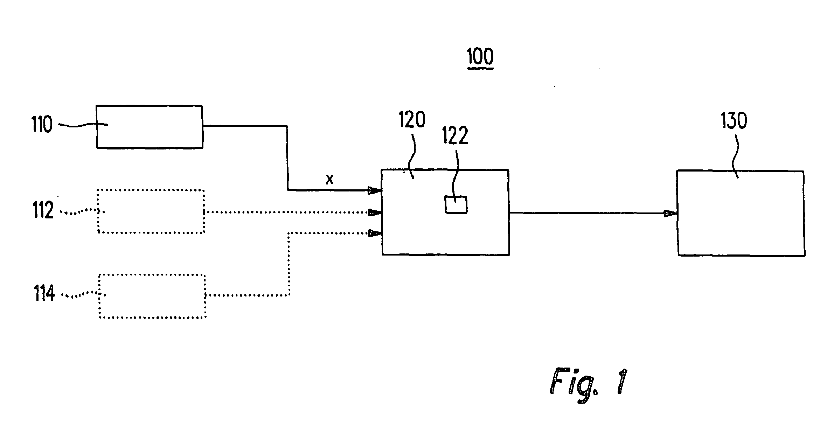

Figur 1 zeigt ein Steuergerät 100 zum Durchführen des erfindungsgemäßen Verfahrens zum Erkennen einer Unaufmerksamkeit des Fahrers eines Fahrzeugs, insbesondere eines Kraftfahrzeugs. Das Steuergerät ist vorzugsweise in dem Fahrzeug (nicht gezeigt) montiert und umfasst einen Lenkradwinkelsensor 110 zum Erfassen des aktuellen Lenkradwinkels x, das heißt der Lenkbewegung, verursacht durch den Fahrer. Darüber hinaus umfasst das Steuergerät 100 eine Steuereinrichtung 120, welche vorzugsweise als Mikrocontroller ausgebildet ist. Die Steuereinrichtung 120 erfasst ein von dem Lenkradwinkelsensor 110 erzeugtes Sensorsignal, welches den Lenkradwinkel x repräsentiert.

Der Lenkradwinkel x repräsentiert einen ersten und erfindungsgemäß bevorzugten Indikator für eine Unaufmerksamkeit des Fahrers. Neben dem Lenkradwinkel kann die Steuereinrichtung 120 grundsätzlich auch noch weitere Sensorsignale von anderen Sensoren 112, 114 als weitere Indikatoren für die Unaufmerksamkeit des Fahrers empfangen und auswerten. Solche weiteren Sensorsignale sollen zunächst unberücksichtigt bleiben, sie finden aber weiter unten in der Beschreibung Erwähnung.

Zum Erkennen einer Unaufmerksamkeit des Fahrers läuft auf der Steuereinrichtung 120 ein Computerprogramm 122 ab, welches die Unaufmerksamkeit gemäß einem erfindungsgemäßen und nachfolgend beschriebenen Verfahren durch Auswerten des Lenkradwinkels x als bevorzugtem Indikator erkennt. Wird eine Unaufmerksamkeit des Fahrers festgestellt, so ist es vorteilhaft, wenn die Steuereinrichtung 120 eine Warneinrichtung 130 ansteuert, damit diese einen akustischen oder optischen Warnhinweis an den Fahrer ausgibt. Aufgrund des Warnhinweises wird dem Fahrer sein unaufmerksames Verhalten beim Führen des Fahrzeugs vergegenwärtigt und ihm Gelegenheit gegeben, seine Aufmerksamkeit wiederherzustellen.

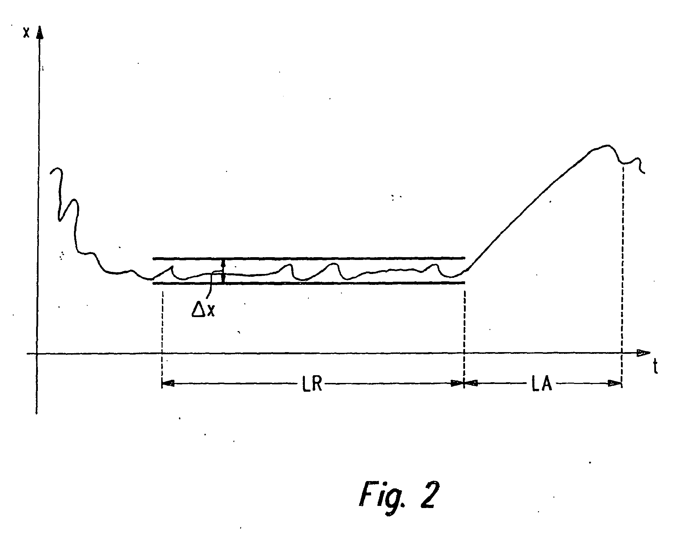

In Figur 2 ist ein typischer Verlauf des Lenkradwinkels aufgezeigt, wie er vorliegt, wenn eine Unaufmerksamkeit des Fahrers mit Hilfe der vorliegenden Erfindung erkannt wird. Dieser Verlauf ist insofern typisch für das Vorliegen einer Unaufmerksamkeit des Fahrers, als dass er zunächst eine Lenkruhephase LR aufweist, in welcher er sich nicht wesentlich ändert; in Figur 2 verbleibt der Lenkwinkel x während der Lenkruhephase LR in dem durch die beiden parallelen horizontalen Linien begrenzten Auslenkungsbereich $\Delta$x. Charakteristisch für das Vorliegen einer Unaufmerksamkeit im Sinne der Erfindung ist dann eine sehr starke beziehungsweise heftige Lenkaktion, die sich an diese Lenkruhephase anschließt. Diese heftige Lenkaktion LA ist in Figur 2 durch den steilen Anstieg des Lenkwinkels x am Ende der Ruhephase repräsentiert.

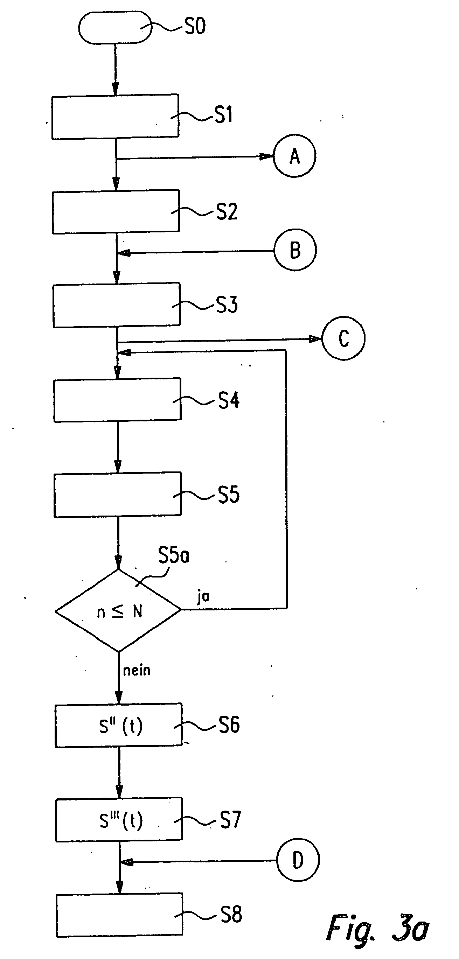

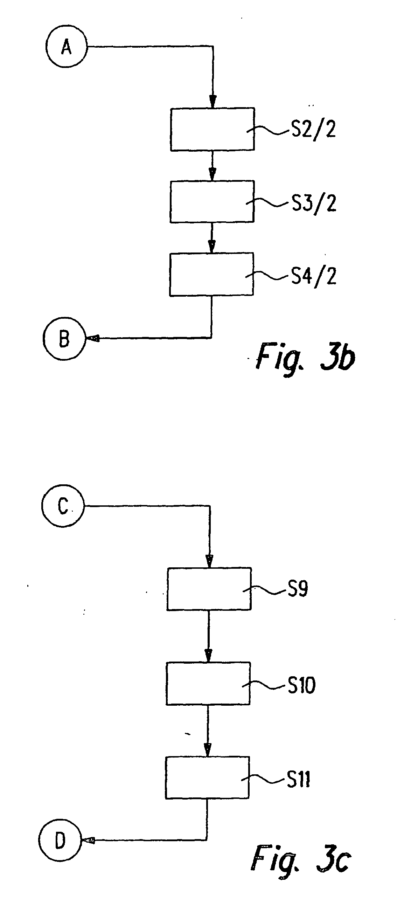

Figur 3 veranschaulicht das mit Hilfe des oben beschrieben Steuergerätes 100 realisierte, Verfahren. Die in Figur 3a aufgezeigten Verfahrensschritte S0 - S8 repräsentieren zunächst ein erstes Ausführungsbeispiel für dieses Verfahren. Dieses wird nachfolgend zunächst durchgehend und zusammenhängend beschrieben. Für dieses erste Ausführungsbeispiel sind die in Figur 3a gezeigten Abzweigungen A, B, C und D unbeachtlich; sie werden erst relevant bei der Beschreibung eines zweiten Ausführungsbeispiels für das erfindungsgemäße Verfahren, welche sich an die Beschreibung des ersten Ausführungsbeispiels anschließt.

Erstes Ausführungsbeispiel

Nach einem Startschritt S0 sieht das erste Ausführungsbeispiel gemäß Figur 3a zunächst die Erfassung einer Lenkbewegung des Lenkrades des Fahrzeugs, das heißt die Erfassung des Lenkverhaltens des Fahrers in Form des Lenkradwinkels x vor (Verfahrensschritt S1). Auf Basis des erfassten Lenkradwinkels x erfolgt dann in einem zweiten Schritt S2 das Erkennen der Lenkruhephase LR, siehe Figur 2. Ebenfalls in Schritt 2 erfolgt dann die Berechnung eines Varianzverhältnisses vv(x, $t_1$) als Quotient einer zweiten zu einer ersten Lenkradwinkelvarianz. Dabei berechnet sich die erste Lenkradwinkelvarianz $v(x, t_1 - \Delta t)$ zu einem frühen Zeitpunkt $t_1 - \Delta t$ gemäß folgender Formel (1):

$$v(x, t_1 - \Delta t) = \mathrm{var}(x(t_1 - \Delta t), ..., x(t_1 - \Delta t - T)) = \frac{1}{T} \sum_{t=(t_1 - \Delta t)}^{(t_1 - \Delta t - T)} (x(t) - \bar{x})^2 \qquad (1)$$

wobei

$x(t_1 - \Delta t)$     den Lenkradwinkel x zum Zeitpunkt $t_1 - \Delta t$;

$\Delta t$     ein Vielfaches des Abtastintervalls;

$T$     ein Beobachtungszeitfenster;

$t_1 - \Delta t$     den Beobachtungszeitpunkt;

$\bar{x}$     einen zeitlichen Mittelwert des Lenkradwinkels x gemittelt über dem Beobachtungszeitfenster T; und

var     die mathematische Varianzfunktion repräsentiert.

Die zweite Lenkradwinkelvarianz $v(x, t_1)$ berechnet sich gemäß folgender Formel (2):

$$v(x, t_1) = \mathrm{var}(x(t_1), ..., x(t_1 - T)) = \frac{1}{T} \sum_{t=(t_1)}^{(t_1 - T)} (x(t) - \bar{x})^2 \qquad (2),$$

wobei die Variablen die gleiche Bedeutung wie bei Formel (1) haben mit dem einzigen Unterschied, dass sie zum Beobachtungszeitpunkt $t_1$ betrachtet werden.

Aus der ersten und zweiten Lenkradwinkelvarianz berechnet sich dann das Varianz-Verhältnis vv(x,$t_1$) wie folgt:

$$vv(x, t_1) = \frac{v(x, t_1)}{v(x, t_1 - \Delta t)} \qquad (3)$$

Das im Verfahrensschritt S2 berechnete Varianz-Verhältnis repräsentiert deswegen ein zuverlässiges Maß für die Schwere der Unaufmerksamkeit des Fahrers beim Lenken des Fahrzeugs zum Zeitpunkt $t_1$, weil es das typische Lenkverhalten eines Fahrers bei Unaufmerksamkeit wirkungsvoll erfasst. Typisches Lenkverhalten bei Unaufmerksamkeit zeichnet sich - wie bereits oben unter Bezugnahme auf Figur 2 gesagt - durch eine erste Lenkruhephase LR ohne oder mit nur geringer Lenkaktivität und durch eine anschließende zweite Lenkaktionsphase LA mit überdurchschnittlich heftigen Lenkbewegungen aus. Die erste Phase führt zu dem frühen Beobachtungszeitpunkt $t_1 - \Delta t$ zu einem geringen Betrag der ersten Varianz. Dagegen führt die zweite Phase, insbesondere zum Zeitpunkt $t_1$, zu einem erheblich größeren Wert für die zweite Varianz. Insgesamt resultiert daraus ein kleiner Wert für den Nenner und ein großer Wert für den Zähler, so dass aufgrund dieser beiden Effekte insgesamt ein großer Wert für das Varianz-Verhältnis gebildet wird. Solange das Varianz-Verhältnis $\leq 1$ ist, deutet dies darauf hin, dass der Fahrer nicht unaufmerksam ist. Erst wenn das Varianz-Verhältnis einen Wert größer 1 annimmt, deutet dies darauf hin, dass der Fahrer des Fahrzeugs dem Straßenverkehr nicht genügend Aufmerksamkeit widmet.

Der Wert des Varianz-Verhältnisses kann theoretisch beliebig groß werden. Beliebig große Werte dieser Art sind jedoch ungeeignet, um die Unaufmerksamkeit des Fahrers hinreichend genau klassifizieren zu können.

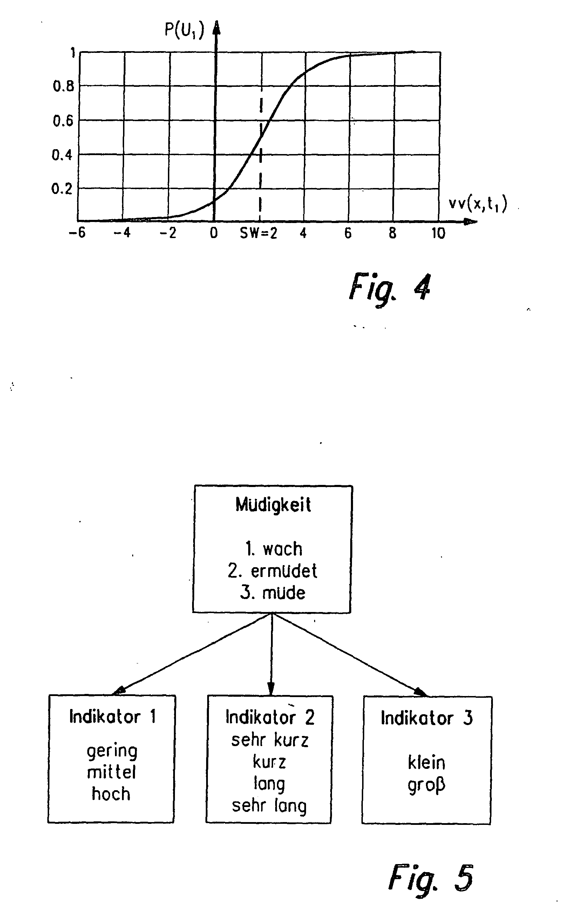

In einer besonderen Variante des Verfahrens wird deshalb in einem nachfolgenden Verfahrensschritt S3 das Varianz-Verhältnis in einen Wahrscheinlichkeitswert umgerechnet. Diese Umrechnung erfolgt vorzugsweise mit Hilfe einer in Figur 4 dargestellten Sigmoid-Funktion mit einem geeignet vorgegebenen Schwellenwert SW. Bei der in Figur 3 dargestellten Sigmoid-Funktion beträgt dieser Schwellenwert SW = 2. Dies bedeutet, dass bei einem auf der Abszisse in Figur 3 aufgetragenen Varianz-Verhältnis $vv(x, t_1)$ von 2 eine Wahrscheinlichkeit $P(U_n)$ mit $n = 1$ (erster Indikator) von 0,5, das heißt von 50 %, dafür besteht, dass der Fahrer des Fahrzeugs zum Zeitpunkt $t_1$ unaufmerksam war. Wie aus dem Graphen der Sigmoid-Funktion in Figur 3 zu erkennen ist, kann die Wahrscheinlichkeit $P(U_1)$ grundsätzlich je nach Größe des Varianz-Verhältnisses einen Wert zwischen 0 und 100 % annehmen.

Die Sigmoid-Funktion gewährleistet einen "weichen" Übergang von totaler Aufmerksamkeit entsprechend einem Wahrscheinlichkeitswert für die momentane Unaufmerksamkeit von 0 zu totaler Unaufmerksamkeit, entsprechend einem Wert für die Unaufmerksamkeit von 1 = 100 %. Mathematisch berechnet sich die in Figur 3 dargestellte Sigmoid-Funktion gemäß folgender Formel (4) :

$$P(U_1) = \frac{1}{1 + e^{-(vv(x, t_1) - S)}} \tag{4},$$

wobei

$P(U_1)$  die Wahrscheinlichkeit für die Unaufmerksamkeit des Fahrers beim Lenken des Fahrzeugs repräsentiert, wobei
$U_1$  ein mit Hilfe des ersten Indikators (Lenkradwinkel: $n = 1$) detektiertes Unaufmerksamkeitsereignis und
$S$  den Schwellenwert

repräsentiert.

Die bisher, das heißt bis einschließlich Verfahrensschritt S3, vorgenommenen Auswertungen des Lenkradwinkels x haben eine Erkenntnis über das Vorhandensein oder Nichtvorhandensein einer Unaufmerksamkeit des Fahrers zum Zeitpunkt $t_1$ ermöglicht. Eine weitere Auswertung der bisher gewonnenen Erkenntnisse, vorzugsweise mit Hilfe eines dynamischen probabilitischen Modells, ermöglicht darüber hinaus Rückschlüsse auf die möglichen Ursachen einer festgestellten Unaufmerksamkeit. Mit den nachfolgend beschriebenen Verfahrensschritten S4 - S6 wird deshalb ein Verfahren aufgezeigt, mit dessen Hilfe beispielsweise festgestellt werden kann, mit welcher Wahrscheinlichkeit Müdigkeit die Ursache für die bei dem Fahrer detektierte Unaufmerksamkeit sein kann.

Diesen soeben beschriebenen Zusammenhang zwischen der Müdigkeit als Ursache für eine daraus resultierende Unaufmerksamkeit beim Lenken des Fahrzeugs ist in Figur 5 veranschaulicht. Die dort eingezeichneten Pfeile zeigen von der Ursache Müdigkeit hin auf verschiedene mögliche Wirkungen, insbesondere eine Unaufmerksamkeit beim Lenken des Fahrzeugs (Indikator 1). Neben der Wirkung Unaufmerksamkeit kann die Müdigkeit jedoch auch noch andere beobachtbare Wirkungen wie zum Beispiel ein gehäuft auftretendes Lidschlussverhalten (Indikator 2) oder eine verzögerte Reaktionsfähigkeit (Indikator 3) haben.

Um aus einem detektierten Unaufmerksamkeitsereignis beim Lenken des Fahrzeugs auf eine Müdigkeit des Fahrers rückschließen zu können, wird in Verfahrensschritt S4 ein erster Wahrscheinlichkeitsvektor $O_{n=1}$ ermittelt, dessen Elemente $O_{n=1,kn=1}$ jeweils Wahrscheinlichkeitswerte dafür repräsentieren, dass der Wahrscheinlichkeitswert $P(U_1)$ in einzelnen vorgegebenen und geeignet ausgewählten Ausprägungsstufen $k_n$ mit $k_n \in \{1...K\}$ auftritt. Der Parameter n repräsentiert dabei einen jeweils ausgewerteten Indikator, wobei $n = 1$ das Lenkverhalten beziehungsweise den Lenkradwinkel als Indikator repräsentiert.

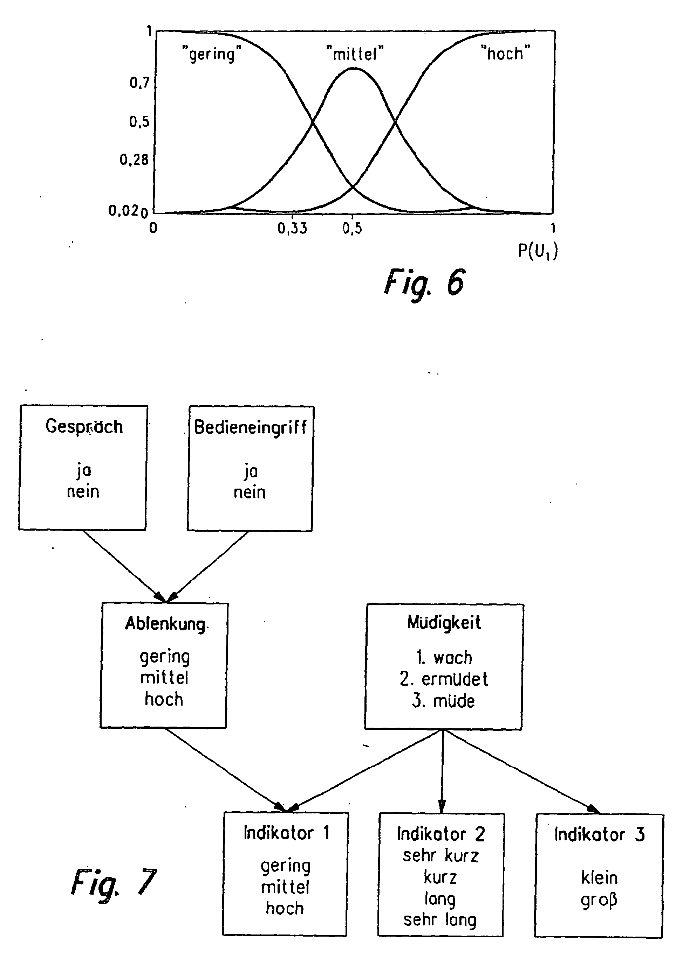

Die Ermittlung des ersten Wahrscheinlichkeitsvektors $O_{n=1}$ ist in Figur 6 veranschaulicht. Dort ist auf der Abszisse der in dem vorherigen Verfahrensschritt S3 ermittelte Wahrscheinlichkeitswert $P(U_1)$ zwischen 0 und 100 % aufgetragen, welcher die Wahrscheinlichkeit für die Unaufmerksamkeit des Fahrers beim Lenken des Fahrzeugs zum Zeitpunkt $t_1$ repräsentiert. Im Verfahrensschritt S4 soll nun der zuvor ermittelte Wahrscheinlichkeitswert $P(U_1)$ vorgegebenen sein und geeignet definierten Ausprägungsstufen $k_1 \in \{1...K_1\}$ zugeordnet werden. Bei dem in Figur 6 gezeigten Ausführungsbeispiel wurden drei Ausprägungsstufen "gering" ($k_1 = 1$), "mittel" ($k_1 = 2$) und "hoch" ($k_1 = 3$) (vergleiche Figur 5) für den Indikator 1 vorgegeben, die in Figur 6 jeweils durch eine eigene Gauß-Funktion repräsentiert sind. Die Anzahl der Ausprägungsstufen für den Indikator 1 ist in diesem Beispiel somit $K_1 = 3$.

Diese Gauß-Funktionen für die jeweiligen Ausprägungsstufen sind hinsichtlich ihrer Anzahl und Parameter, wie zum Beispiel ihrer Mittelpunkte oder ihrer Varianzen, je nach Anwendungsfall geeignet zu parametrieren. Wie aus Figur 6 ersichtlich, würde nach Durchführung des Verfahrensschrittes S4 einem beispielhaft angenommenen Wahrscheinlichkeitswert $P(U_1)$ von 0,33 für die Unaufmerksamkeit des Fahrers eine Wahrscheinlichkeit von 70 % dafür zugeordnet, dass dieser Wahrscheinlichkeitswert mit nur geringer Ausprägung auftritt. Mit 28 %-iger Wahrscheinlichkeit tritt der berechnete Wahrscheinlichkeitswert mit einer mittleren Ausprägung auf und mit lediglich 2 % tritt der berechnete Wahrscheinlichkeitswert mit einer hohen Ausprägung auf.

Anders ausgedrückt erhält man bei dem in Figur 6 gezeigten Beispiel für den angenommenen Wahrscheinlichkeitswert von 0,33 für die Unaufmerksamkeit des Fahrers zum Zeitpunkt $t_1$ die Aussage, dass die Wahrscheinlichkeit für eine Unaufmerksamkeit des Fahrers mit einer Wahrscheinlichkeit von 70 % nur gering ausgeprägt war, das heißt dass der Fahrer mit einer Wahrscheinlichkeit von 70 % aufmerksam war. Gleichermaßen erhält man die Aussage, dass der Fahrer mit einer Wahrscheinlichkeit von 28 % mittelmäßig aufmerksam war und dass er mit einer Wahrscheinlichkeit von lediglich 2 % hochgradig unaufmerksam war.

In einem nachfolgenden Verfahrensschritt S5 können nun eine beliebige Anzahl von Müdigkeitsstufen definiert werden und bedingte Wahrscheinlichkeiten zwischen diesen Stufen sowie dem beobachteten Lenkunaufmerksamkeitsereignis zugeordnet werden. Bei dem in Figur 5 gezeigten Beispiel sind insgesamt drei Müdigkeitsstufen "wach", "ermüdet" und "müde" vorgesehen. Es können dann, ebenfalls noch in Verfahrensschritt S5, bedingte Wahrscheinlichkeiten in Form einer Matrix B zwischen jeder dieser Müdigkeitsstufen und einer der oben beschriebenen Ausprägungsstufen zugeordnet werden. Diese Matrix B umfasst eine Gesamtzahl von Matrixelementen, welche sich aus dem Produkt Anzahl der Ausprägungsstufen multipliziert mit der Anzahl der vorgegebenen Müdigkeitsstufen berechnet. Bei drei Ausprägungsstufen "gering", "mittel" und "hoch" für das Lenkunaufmerksamkeitsereignis und bei den oben genannten drei Müdigkeitsstufen ergibt sich für die Matrix B eine Gesamtanzahl von 3 * 3 = 9 bedingten Wahrscheinlichkeiten. Eine dieser Wahrscheinlichkeiten gibt beispielsweise an, wie groß die Wahrscheinlichkeit dafür ist, dass ein Lenkunaufmerksamkeitsereignis in der "hohen" Ausprägungsstufe auftritt, gegeben, dass das probabilistische Modell in der ersten Müdigkeitsstufe "wach" ist. Diese bedingten Wahrscheinlichkeiten sind geeignet zu parametrieren.

Mit dem in Schritt S5 ermittelten Wahrscheinlichkeitsvektor $O_{n=1}$ und der berechneten Matrix B ist es nun möglich, in einem Verfahrensschritt S6 einen Müdigkeitswahrscheinlichkeitsvektor S' zu ermitteln, dessen Elemente jeweils Wahrscheinlichkeiten P (Müdigkeitsstufe) dafür repräsentieren, dass die erfasste Unaufmerksamkeit des Fahrers beim Lenken des Fahrzeugs einzelnen vorgegebenen und geeignet ausgewählten Müdigkeitsstufen zugeordnet ist. Die Berechnung des Müdigkeitswahrscheinlichkeitsvektors S' erfolgt gemäß folgender Formel (5):

$$S'(t) = O_1^T \cdot B_1 \qquad\qquad (5),$$

wobei

$O^T_{n=1}$ die Transponierte des ersten Wahrscheinlichkeitsvektors; und
$B_1$ die Matrix B für den erfassten Indikator für Lenkunaufmerksamkeiten, repräsentiert durch den Index 1

repräsentiert.

Die soeben beschriebene Rechenvorschrift (5) zur Berechnung des Müdigkeitswahrscheinlichkeitsvektors S' hat den Nachteil, dass sie lediglich auf einer Auswertung des Lenkradwinkels x als Indikator (n=1) basiert. Andere mögliche beobachtbare Auswirkungen von Müdigkeit, wie sie in Figur 5 durch die Indikatoren 2 (zum Beispiel Lidschlusshäufigkeit) oder Indikator 3 (zum Beispiel Reaktionszeit) angedeutet sind, werden in Formel 5 zur Berechnung des Müdigkeitswahrscheinlichkeitsvektors nicht berücksichtigt.

Es ist jedoch auch möglich, diese Indikatoren 2 und 3 sowie weitere geeignete Indikatoren n, wie zum Beispiel den Gierwinkel des Fahrzeugs, das Abstandsverhalten zum Vorderfahrzeug oder ein Abkommen von einer Fahrspur, sofern messbar, für die Berechnung eines präzisierten Müdigkeitswahrscheinlichkeitsvektors S'' heranzuziehen. Die soeben beschriebenen Verfahrensschritte S4 und S5 sind dann jeweils nicht nur für den beobachteten Lenkwinkel als Indikator n = 1, sondern auch für die weiteren gewünschten Indikatoren wie Lidschlussverhalten (n = 2) und/oder Reaktionszeit (n = 3) etc. separat durchzuführen (Verfahrensschritt S5a). Im Rahmen von Verfahrensschritt S4 sind dann jeweils individuell für jeden Indikator n eine individuelle Anzahl von Ausprägungsstufen $k_n$ mit $k_n$ = 1...K festzulegen. Diese Anzahl der Ausprägungsstufen $k_n$ entspricht dann jeweils der Anzahl der Elemente eines dem jeweiligen Indikator zugeordneten Wahrscheinlichkeitsvektors $O_n$. Diese Elemente $O_{n,kn}$ repräsentieren jeweils Wahrscheinlichkeiten P$(O_{n\_,kn})$ dafür, dass Wahrscheinlichkeitswerte P$(U_n)$ für die anderen Unaufmerksamkeitsindikatoren n = 2...N neben der Lenkunaufmerksamkeit n = 1 in den einzelnen für die Indikatoren individuell vorgegebenen und geeignet ausgewählten Ausprägungsstufen $k_n$ auftreten.

Auch für diese weiteren Indikatoren n ist dann entsprechend Verfahrensschritt S5 wieder eine entsprechende Matrix $B_n$ individuell vorzugeben. Aufgrund der dann verfügbaren Daten berechnet sich der präzisierte Müdigkeitswahrscheinlichkeitsvektor S'' in Schritt S6 dann gemäß folgender Formel (6):

$$S''(t) = \prod_{n=1}^{N} O_n^T \cdot B_n \qquad (6),$$

wobei:

n    den n-ten Indikator für die Unaufmerksamkeit des Fahrers

$k_n$    das k'te Element des Vektors $O_n$ beziehungsweise die k'te Ausprägungsstufe für den Indikator n;

$O_n^T$    die Transponierte eines Wahrscheinlichkeitsvektors;

$B_n$    die Matrix von bedingten Wahrscheinlichkeiten zwischen einzelnen vorgegebenen Müdigkeitsstufen und einem durch den Indikator n indizierten Unaufmerksamkeitsereignis; und

N    die Anzahl der verwendeten Indikatoren repräsentiert.

Bei den bisher beschriebenen beiden Varianten zur Berechnung des Müdigkeitswahrscheinlichkeitsvektors S wurde lediglich von der Schwere eines detektierten Unaufmerksamkeitsereignisses, sei es aufgrund eines detektierten Lenkunaufmerksamkeitsereignisses oder auch aufgrund von zusätzlich detektieren Ereignissen, wie erhöhtem Lidschlussverhalten oder verkürzter Reaktionszeit, auf die am wahrscheinlichsten vorliegende Müdigkeitsstufe rückgeschlossen. Eine weitere Präzisierung dieses Rückschlusses von der beobachteten Unaufmerksamkeit zurück auf eine vorliegende Müdigkeitsstufe als Ursache für diese Unaufmerksamkeit lässt sich dadurch erzielen, dass neben der Schwere des Unaufmerksamkeitsereignisses zusätzlich auch die in einem Zeitschritt zuvor ermittelte wahrscheinlichste Müdigkeitsstufe berücksichtigt wird. Auf diese Weise wird gewährleistet, dass nicht bereits ein einmalig beziehungsweise erstmalig detektiertes Unaufmerksamkeitsereignis sofort zu einem Rückschluss auf eine hohe Müdigkeitsstufe führt. Dies würde der realen Charakteristik von Müdigkeit insofern widersprechen, als dass Müdigkeit grundsätzlich ein Phänomen ist, welches nicht plötzlich auftritt, sondern sich erst im Laufe einer gewissen Zeitdauer langsam aufbaut.

Bei den oben erwähnten und in Figur 5 beschriebenen drei Müdigkeitsstufen sind deshalb die Wechsel von einer Müdigkeitsstufe zu einer anderen mit unterschiedlich bedingten Wahrscheinlichkeiten versehen. Diese bedingten Wahrscheinlichkeiten werden im Rahmen des probabilistischen Modells vorzugsweise in Form einer Matrix A geeignet vorgegeben. Die bedingten Wahrscheinlichkeiten in der Matrix A sollten insbesondere zum Ausdruck bringen, dass ein direkter Übergang zum Beispiel von Müdigkeitsstufe 1 "wach" nach Müdigkeitsstufe 3, "müde" wesentlich unwahrscheinlicher ist als ein direkter Übergang von Stufe 1 nach Stufe 2, "ermüdet", bei der gegenüber Stufe 1 bereits erste Einbußen hinsichtlich der Aufmerksamkeit des Fahrers zu erkennen sind. Die Parametrierung der Matrix A sollte außerdem berücksichtigten, dass die Übergänge von einem wachen in einen Zustand mit größerer Müdigkeit mit anderen bedingten Wahrscheinlichkeiten erfolgen, wie die Übergänge von einem müden in einen wacheren Zustand.

Unter zusätzlicher Berücksichtigung der im Zeitschritt zuvor ermittelten wahrscheinlichsten Müdigkeitsstufe berechnet sich der dadurch nochmals präzisierte Müdigkeitswahrscheinlichkeitsvektor S''' dann im Schritt S7 gemäß der folgenden rekursiven Formel (7):

$$S'''(t_1) = S''(t_1) \cdot A \cdot S'''(t_1 - 1) \qquad (7),$$

wobei

S''$(t_1)$    den präzisierten Müdigkeitsvektor S'' ohne Berücksichtigung der im Zeitschritt zuvor ermittelten wahrscheinlichsten Müdigkeitsstufe;

A          die Matrix der bedingten Wahrscheinlichkeiten zwischen einer Müdigkeitsstufe im letzten Zeitschritt und einer aktuellen Müdigkeitsstufe; und

$S'''(t_1 - 1)$          den präzisierten Müdigkeitsvektor $S'''$ im Zeitschritt $t_1 - 1$

repräsentiert.

Zum Starten der rekursiven Berechnung des präzisierten Müdigkeitswahrscheinlichkeitsvektors $S'''$ gemäß Formel (7) als dritte Variante empfiehlt sich ein Anfangswert von $S'''(0) = (1, 0, 0)^T$.

Anders ausgedrückt bewirkt die präzisere Berechnung des Müdigkeitswahrscheinlichkeitsvektors $S'''$ gemäß Formel (7) eine Glättung des Müdigkeitsvektors über die Zeit beziehungsweise er verhindert, dass ein erstmalig erkanntes Unaufmerksamkeitsereignis sofort zu einer sprunghaften Änderung der als wahrscheinlichste Ursache für das detektierte Unaufmerksamkeitsereignis vermuteten Müdigkeitsstufe führt. Durch geeignete Parametrierung von insbesondere der Matrix A wird weiterhin sichergestellt, dass umgekehrt eine plötzlich festgestellte große Aufmerksamkeit des Fahrers nicht sofort zu einer niedrigen Müdigkeitsstufe führt, wenn unmittelbar zuvor noch eine sehr hohe Müdigkeitsstufe als wahrscheinliche Ursache für eine detektierte Unaufmerksamkeit festgestellt wurde.

Das bisher beschriebene Verfahren zielte stets darauf ab, aufgrund einer erfassten Lenkunaufmerksamkeit sowie optional auch aufgrund weiterer Indikatoren für eine Unaufmerksamkeit des Fahrers auf eine gewisse Müdigkeitsstufe als Ursache für die erfasste Unaufmerksamkeit rückzuschließen. Dies ist in Figur 7 nochmals grafisch veranschaulicht, wo die Pfeile von einer Ursache zu einer Wirkung zeigen. In Figur 7 ist jedoch aufgrund der Pfeilführung auch zu erkennen, dass die Müdigkeit nach dem zugrunde liegenden Modell zwar alleinige Ursache für die Indikatoren 2, das heißt Lidschlussverhalten, und 3, das heißt verkürzte Reaktionszeit, ist, dass sie aber nicht die einzige Ursache für eine detektierte Lenkunaufmerksamkeit sein muss. Vielmehr kann ergänzend oder alternativ zur Müdigkeit auch eine gegebene temporäre Ablenkung des Fahrers Ursache für eine bei ihm detektierte Lenkunaufmerksamkeit sein. Ursache für eine Ablenkung kann wiederum ein zum Beispiel mit dem Beifahrer geführtes Gespräch oder ein vorgenommener Eingriff in ein Bedienelement, zum Beispiel das Radio oder das Handschuhfach des Fahrzeugs sein. Werden neben den bisher genannten Unaufmerksamkeitsereignissen oder Indikatoren zusätzlich auch mögliche Ursachen für eine Ablenkung mit Hilfe geeigneter Sensoren, zum Beispiel eines Mikrofons 112 oder einer Kamera 114 (siehe Figur 1) erfasst, so kann durch Auswertung dieser erfassten Ereignisse mit Hilfe des probabilistischen Modells eine Aussage darüber getroffen werden, mit welcher Wahrscheinlichkeit eine Ablenkung des Fahrers, zum Beispiel aufgrund eines geführten Gespräches oder eines vorgenommenen Bedieneingriffs, und mit welcher Wahrscheinlichkeit Müdigkeit als Ursache für die beobachtete Unaufmerksamkeit angenommen werden kann.

Die mit einer der Varianten des ersten Ausführungsbeispiels detektierte Unaufmerksamkeit des Fahrers wird schließlich mit einem Fehlerkriterium, insbesondere einem jeweils geeignet vorgegebenen Schwellenwert verglichen, um dann nach Maßgabe durch das Ergebnis dieses Vergleiches ein Warnsignal an den Fahrer auszugeben (Verfahrensschritt S8 gemäß Figur 3a).

Zweites Ausführungsbeispiel

Das zweite erfindungsgemäße Ausführungsbeispiel des Verfahrens ist zwar grundsätzlich unabhängig von dem ersten Ausführungsbeispiel, es weist jedoch einzelne Verfahrensschritte auf, welche identisch sind mit einzelnen Verfahrensschritten aus dem ersten Ausführungsbeispiel. Ein grundsätzlicher Unterschied zwischen dem ersten und zweiten Ausführungsbeispiel besteht darin, dass bei dem ersten Ausführungsbeispiel das Erkennen einer Lenkaktion LA und die Berechnung eines Verknüpfungsergebnisses zwischen der Ausprägung der Lenkruhephase und der Ausprägung der Lenkaktion in Form der Berechnung des Varianzverhältnisses zusammenfallen. Das zweite Ausführungsbeispiel bietet demgegenüber den Vorteil, dass nicht nur die Lenkruhephase LR, sondern auch die anschließende Lenkaktion LA und die erfindungsgemäße Verknüpfung der Ausprägungen dieser beiden Phasen jeweils als separate Verfahrensschritte getrennt durchgeführt werden können, wie dies nachfolgend unter Bezugnahme auf die Figuren 3a, 3b und 3c näher beschrieben wird.

Nach einem Startschritt S0 sieht das zweite Ausführungsbeispiel gemäß Figur 3a zunächst die Erfassung der Lenkbewegung des Lenkrades des Fahrzeugs, das heißt die Erfassung des Lenkverhaltens des Fahrers in Form des Lenkradwinkels x vor (Verfahrensschritt S1); insoweit stimmen das erste und das zweite Ausführungsbeispiel noch überein. Für das zweite Ausführungsbeispiel folgt nun nicht der Verfahrensschritt S2 gemäß Figur 3a, sondern das Verfahren verzweigt über die Marke A in die Figur 3b, wo in Verfahrensschritt S2/2 zunächst die Ausprägung der Lenkruhephase ermittelt wird. Die Ausprägung der Lenkruhephase meint insbesondere deren Zeitdauer. Eine Lenkruhephase ist so lange gegeben, so lange der Lenkwinkel des Fahrzeugs innerhalb des vorbestimmten Lenkradwinkelintervalls $\Delta x$ liegt, vgl. Figur 2. Die Zeitdauer, während der diese Situation andauert, repräsentiert dann die Ausprägung der Lenkruhephase LR.

Nachfolgend wird in einem Verfahrensschritt S3/2 die Ausprägung einer sich an die detektierte Lenkruhephase anschließenden Lenkaktion detektiert. Dazu wird der dann auftretende maximale Gradient des Lenkwinkels ermittelt.

In Figur 2 ist dieser Gradient in Form der Steigung des Lenkwinkels veranschaulicht, wie er auftritt, nachdem der Lenkradwinkel das Lenkradwinkelintervall Δx verlassen hat.

In Verfahrensschritt S4/2 werden dann die Ausprägung der Lenkruhephase und der Lenkaktion über einen mehrdimensionalen Operator miteinander verknüpft. Bei dem mehrdimensionalen Operator handelt es sich um eine Gewichtungsfunktion. Das Ergebnis dieser Anwendung des mehrdimensionalen Operators repräsentiert dann ein geeignetes Maß für die Schwere der Unaufmerksamkeit des Fahrers beim Lenken des Fahrzeugs. Vorzugsweise wird die Verknüpfung der beiden genannten Ausprägungen jedoch nur dann durchgeführt, wenn sich in den zuvor durchgeführten Schritten S2/2 oder S3/2 gezeigt hat, dass die Ausprägung der Lenkruhephase in Form ihrer Zeitdauer größer ist als eine vorbestimmte Mindestzeitdauer und der maximale Gradient des Lenkradwinkels einen vorbestimmten Gradientenschwellenwert überschreitet. Andernfalls werden die Ausprägungen der Lenkruhephase und der Lenkaktion von dem erfindungsgemäßen Verfahren nicht als hinreichend stark genug ausgeprägt angesehen, um aus ihrem kombinierten Vorliegen auf eine Unaufmerksamkeit des Fahrers schließen zu können.

Der mehrdimensionale Operator wird erfindungsgemäß nach Maßgabe durch die aktuelle Geschwindigkeit des Fahrzeugs und/oder vorzugsweise nach Maßgabe durch die Dynamik des Fahrstils des Fahrers des Fahrzeugs dimensioniert. Konkret bedeutet dies, dass bei der Auswertung der detektierten Ausprägungen der Lenkruhephase und der Lenkaktion berücksichtigt wird, dass die Lenkbewegungen bei hohen Geschwindigkeiten des Fahrzeugs typischerweise geringer sind als bei niedrigen Geschwindigkeiten. Die Anpassung an den Fahrstil des Fahrers verhindert, dass eine zum Beispiel von einem Rallyefahrer absichtlich durchgeführte hektische Lenkbewegung in Verbindung mit einer zuvor festgestellten vermeintlichen Lenkruhephase fälschlicherweise als eine Unaufmerksamkeit des Fahrers interpretiert wird.

Das in Verfahrensschritt S4/2 ermittelte Verknüpfungsergebnis gemäß dem zweiten Ausführungsbeispiel kann nun auf verschiedene Weise weiter ausgewertet und verarbeitet werden.

Eine erste Möglichkeit besteht darin, dass dieses mit Hilfe zum Beispiel der Sigmoid-Funktion normalisiert wird. Diese Möglichkeit ist in Figur 3b durch die Marke B angezeigt, die auf den Eingang von Verfahrensschritt S3 in Figur 3a verzweigt. Es kann dann nicht nur der Verfahrensschritt S3, sondern es können dann auch alle weiteren Verfahrensschritte S4 bis S8 wie oben unter Bezugnahme auf Figur 3a beschrieben, durchgeführt werden.

Eine zweite Möglichkeit zur Weiterverarbeitung des in Verfahrensschritt S4/2 gewonnenen Verknüpfungsergebnisses besteht darin, dass es zwar noch mit Hilfe der Sigmoid-Funktion in Verfahrensschritt S3 normiert wird, dann aber nicht gemäß Verfahrensschritt S4 ff. weiter ausgewertet, sondern wie in Figur 3a durch die Marke C angedeutet, in Verfahrensschritt S9 gemäß Figur 3c weiterverarbeitet wird. Mit diesem Schritt wird angedeutet, dass die zuvor durchgeführte Berechnung des Verknüpfungsergebnisses, sei es gemäß dem ersten Ausführungsbeispiel in Schritt 2 oder gemäß dem zweiten Ausführungsbeispiel in Schritt S4/2, mehrfach während eines vorbestimmten Messzeitintervalls durchgeführt werden sollte. Die wiederholte Durchführung der Berechnung der Verknüpfungsergebnisse zu verschiedenen Zeitpunkt ti mit i = 1-I während des Messzeitintervalls führt dazu, dass am Ende des Messzeitintervalls vorzugsweise eine Vielzahl von Verknüpfungsergebnissen vorliegen. Diese Verknüpfungsergebnisse werden vorzugsweise, wie bisher beschrieben, am Ausgang von Verfahrensschritt S3 abgegriffen, weil sie dann in normierter Form vorliegen. Alternativ können aber auch die unmittelbar in den Verfahrensschritten S2 und S4/2 gewonnenen Verknüpfungsergebnisse direkt in Verfahrensschritt S9 gesammelt und gespeichert werden. In Verfahrensschritt S10 werden diese Verknüpfungsergebnisse dann individuell gewichtet, indem jedem dieser Ergebnisse ein Gewichtungsfaktor zugeordnet wird. Diese Gewichtungsfaktoren repräsentieren die jeweilige Fahrsituation des Fahrzeugs oder die aktuelle Ablenkung des Fahrers jeweils zu dem Zeitpunkt, auf den sich das Verknüpfungsergebnis bezieht.

In Verfahrensschritt S11 wird dann schließlich ein gewichtetes Verknüpfungsergebnis durch mathematische, vorzugsweise arithmetische gewichtete Mittelwertbildung der während des Messzeitintervalls gewonnenen Verknüpfungsergebnisse unter Berücksichtigung von deren zugeordneten Gewichtungsfaktoren berechnet.

Die Gewichtungsfaktoren werden unter Berücksichtigung der Tageszeit, das heißt circadianer Einflussfaktoren und/oder der Zeit seit Fahrtbeginn festgelegt. Mit dem gewichteten Ergebnis der Verknüpfung liegt ein sehr zuverlässiges und vor allen Dingen relativ einfach und schnell zu ermittelndes Maß für die Schwere der Unaufmerksamkeit des Fahrers beim Fahren des Fahrzeugs vor. Dieses gemittelte Ergebnis der Verknüpfung wird dann vorzugsweise wie in Figur 3c in Verbindung mit Figur 3a über die Marke D in Verfahrensschritt S8 einem Fehlerkriterium unterzogen und zur Generierung eines Warnsignals an den Fahrer ausgewertet. Das Fehlerkriterium ist dann erfüllt, wenn die Summe aller in den letzten x Minuten berechneten Verknüpfungsergebnisse jeweils gewichtet mit ihren individuellen Gewichtungsfaktoren einen vorgegebenen Schwellenwert überschreitet.

Die bisher beschriebene zweite Möglichkeit zur Weiterverarbeitung des in Verfahrensschritt S4/2 gemäß dem zweiten Ausführungsbeispiel gewonnenen Verknüpfungsergebnisses durch die Schritte S9 bis S11 besteht gleichermaßen auch für das in Verfahrensschritt S2 gemäß dem ersten Ausführungsbeispiel gewonnenen Verknüpfungsergebnis.

Eine dritte Möglichkeit zur Weiterverarbeitung des Verknüpfungsergebnisses aus Schritt S2 oder aus Schritt S4/2 besteht in einer vorzugsweise zeitlich parallelen Ausführung der Schritte S4 bis S7 und S9 bis S11.

Die beiden Ausführungsbeispiele des Verfahrens in allen ihren Varianten werden vorzugsweise in Form mindestens eines Computerprogramms realisiert. Das Computerprogramm kann gegebenenfalls zusammen mit weiteren Computerprogrammen auf einem Datenträger abgespeichert sein. Bei dem Datenträger kann es sich um eine Diskette, eine Compact Disc, einen sogenannten Flash-Memory oder dergleichen handeln. Das auf dem Datenträger abgespeicherte Computerprogramm kann dann als Produkt an einen Kunden verkauft werden. Alternativ zu einer Übertragung per Datenträger steht auch die Möglichkeit einer Übertragung über ein Kommunikationsnetzwerk, insbesondere das Internet.

**Patentansprüche**

1.  Verfahren zum Erkennen, wann der Fahrer eines Fahrzeugs, insbesondere eines Kraftfahrzeugs, unaufmerksam wird, umfassend folgende Schritte:

    - Erfassen einer Bewegung eines Lenkrades des Fahrzeugs in Form eines Lenkradwinkels x (Schritt S1); und
    - Erkennen einer Lenkruhephase und Ermitteln der Größe der Ausprägung der Lenkruhephase durch Auswerten des erfassten Lenkradwinkels und/oder dessen zeitlicher Änderung,

    wobei die Ausprägung der Lenkruhephase ermittelt wird als diejenige Zeitdauer, während derer der Lenkradwinkel innerhalb eines vorbestimmten Lenkradwinkelintervalls ($\Delta$x) bleibt (Schritt S2/2), wobei das Lenkradwinkelintervall durch die aktuelle Geschwindigkeit des Fahrzeugs vorbestimmt wird (Schritt S2/2); und

    - Erkennen einer sich an die Lenkruhephase anschließenden Lenkaktion und Ermitteln der Größe der Ausprägung der Lenkaktion durch Auswerten der zeitlichen Änderung des Lenkradwinkels (Schritt S3/2); und
    - Erkennen eines Zustandes der Unaufmerksamkeit, wenn sowohl die Lenkruhephase wie auch die anschließende Lenkaktion in einer vorbestimmten Mindestausprägung vorliegen; und
    - Ermittlung eines Maßes für die Schwere der Unaufmerksamkeit durch Bewerten des Ergebnisses einer Verknüpfung der Ausprägung der Lenkruhephase und der Ausprägung der Lenkaktion, wobei die Verknüpfung der Ausprägung der Lenkruhephase und der Ausprägung der Lenkaktion durch ein Kennfeld, eine Gewichtungsfunktion oder eine logische Entscheidungsfunktion erfolgt.

2.  Verfahren nach Anspruch 1,
    **dadurch gekennzeichnet,** und der Ausprägeng der Lenkaktion dass die Ausprägung der Lenkaktion im Anschluss an eine vorangegangene Lenkruhephase in Form des dann auftretenden maximalen Gradienten des Lenkradwinkels ermittelt wird (Schritt S3/3).

3.  Verfahren nach Anspruch 2,
    **dadurch gekennzeichnet,**
    **dass** die vorbestimmte Mindestausprägung von Lenkruhephase und Lenkaktion vorliegt, wenn die Ausprägung der Lenkruhephase in Form ihrer Zeitdauer größer ist als eine vorbestimmte Mindestzeitdauer und der maximale Gradient des Lenkradwinkels einen vorbestimmten Gradientenschwellenwert überschreitet (Schritt S4/2).

4.  Verfahren nach einem der Ansprüche 1-3,
    **dadurch gekennzeichnet,**
    **dass** Verknüpfung der Ausprägung der Lenkruhephase und der Ausprägung der Lenkaktion nach Maßgabe durch die Geschwindigkeit des Fahrzeugs und/oder eine Dynamik des Fahrstils des Fahrers des Fahrzeugs dimensioniert wird.

5.  Verfahren nach einem der Ansprüche 1-4,
    **dadurch gekennzeichnet,**
    **dass** in einem nachfolgenden Schritt (Schritt S3) das Ergebnis der Verknüpfung, insbesondere mittels der Sigmoid-Funktion, auf einen Wahrscheinlichkeitswert $P(U_1)$ zwischen 0 und 100 % abgebildet wird, welcher die Unaufmerksamkeit des Fahrers beim Lenken des Fahrzeugs zum Zeitpunkt $t_1$ repräsentiert.

6.  Verfahren nach Anspruch 5,
    **gekennzeichnet durch**
    folgende weitere Schritte (Schritt S6) zum Beurteilen der Müdigkeit des Fahrers:

- Ermitteln eines ersten Wahrscheinlichkeitsvektors $O_{n=1}$, dessen Elemente $O_{n=1, k1}$ jeweils Wahrscheinlichkeitswerte $P(O_{1,k1})$ dafür repräsentieren, dass ein Wahrscheinlichkeitswert $P(U_1)$ in einzelnen vorgegebenen und ausgewählten Ausprägungsstufen $k_1$ mit $k_l \in \{1...K_1\}$ auftritt (Schritt S4); und

- Ermitteln eines Müdigkeitswahrscheinlichkeitsvektors S', dessen Elemente jeweils Wahrscheinlichkeiten P (Müdigkeitsstufe) dafür repräsentieren, dass die erfasste Unaufmerksamkeit des Fahrers beim Lenken des Fahrzeugs einzelnen vorgegebenen und geeignet ausgewählten Müdigkeitsstufen zugeordnet ist, gemäß folgender Formel (5):

$$S'(t) = O_1^T \cdot B_1; \qquad\qquad (5),$$

wobei

$O_1^T$ die Transponierte des ersten Wahrscheinlichkeitsvektors;

$B_1$ die Matrix B vorgegebene bedingte Wahrscheinlichkeiten bezüglich der Lenkunaufmerksamkeit, repräsentiert **durch** den Indikator n = 1; und

$K_1$ die Anzahl der Ausprägungsstufen für den Indikator n = 1

repräsentiert.

**7.** Verfahren nach Anspruch 6,
**gekennzeichnet durch**
folgende weitere Schritte:

- Ermitteln von weiteren Wahrscheinlichkeitsvektoren $O_{n=2}...O_{n=N}$, deren Elemente $O_{n,kn}$ mit $k_n = 1...K_n$ jeweils Wahrscheinlichkeiten $P(O_{n,kn})$ dafür repräsentieren, dass Wahrscheinlichkeitswerte $P(U_n)$ für andere Unaufmerksamkeitsindikatoren n = 2...N des Fahrers, neben der Lenkunaufmerksamkeit n = 1, insbesondere das Lidschlussverhalten n = 2 oder die Reaktionszeit n = 3, in einzelnen, für die Unaufmerksamkeitsindikatoren individuell vorgegebenen Ausprägungsstufen $k_n$ auftreten, und

- sich der Müdigkeitswahrscheinlichkeitsvektor S'' (Schritt S6) dann gemäß folgender Formel (6) berechnet:

$$S''(t) = \prod_{n=1}^{N} O_n^T \cdot B_n \qquad\qquad (6),$$

wobei

n den n'ten Indikator für die Unaufmerksamkeit des Fahrers;

$O_n^T$ die Transponierte der weiteren Wahrscheinlichkeitsvektoren;

$B_n$ die Matrix B für den Indikator n; und
N die Anzahl der Indikatoren

repräsentiert.

**8.** Verfahren nach einem der Ansprüche 6 oder 7,
**gekennzeichnet durch**

- Speichern des Müdigkeitswahrscheinlichkeitsvektors S'''(t-1); und
- Berechnen eines weiterhin präzisierten Müdigkeitswahrscheinlichkeitsvektors S'''(t) gemäß folgender Formel (7) (Schritt S7) :

$$S'''(t) = S''(t) \cdot A \cdot S'''(t-1), \qquad\qquad (7)$$

wobei

A die Matrix der bedingten Wahrscheinlichkeiten zwischen einer Müdigkeitsstufe aus dem letzten Zeitschritt und einer aktuellen Müdigkeitsstufe

repräsentiert.

9.  Verfahren nach einem der Ansprüche 1 bis 8,
    **dadurch gekennzeichnet,**
    **dass** neben der Lenkunaufmerksamkeit und den optionalen weiteren Indikatoren für die Unaufmerksamkeit des Fahrers auch festgestellt wird, ob der Fahrer ein Gespräch führt oder einen Eingriff in ein Bedienelement, zum Beispiel das Radio oder das Handschuhfach des Fahrzeugs tätigt; und dass durch Auswertung dieser erfassten Ereignisse mit Hilfe eines probabilistischen Modells eine Aussage darüber getroffen werden kann, mit welcher Wahrscheinlichkeit eine Ablenkung des Fahrers aufgrund des Gesprächs oder des Bedieneingriffs und mit welcher Wahrscheinlichkeit Müdigkeit des Fahrers als Ursache für die beobachtete Unaufmerksamkeit angenommen werden kann.

10.  Verfahren nach einem der Ansprüche 1 bis 5,
     **gekennzeichnet , durch** folgende Schritte:

     - Durchführen der Verknüpfung zu verschiedenen Zeitpunkten ti mit i=1-I während eines vorbestimmten Messzeitintervalls;
     - Speichern der Ergebnisse der Verknüpfungen zu den Zeitpunkten ti jeweils zusammen mit zugeordneten Gewichtungsfaktoren, welche die Fahrsituation des Fahrzeugs oder die aktuelle Ablenkung des Fahrers jeweils zum Zeitpunkt ti repräsentieren; und
     - Berechnen eines gewichteten Ergebnisses der Verknüpfung **durch** mathematische, vorzugsweise arithmetische Mittelwertbildung der während des Messzeitintervalls gespeicherten Ergebnisse unter Berücksichtigung von deren zugeordneten Gewichtungsfaktoren.

11.  Verfahren nach Anspruch 10,
     **dadurch gekennzeichnet,**
     **dass** die Gewichtungsfaktoren unter Berücksichtigung circadianer Einflussfaktoren und/oder der Zeit seit Fahrtbeginn berechnet werden.

12.  Verfahren nach Anspruch 10 oder 11,
     **gekennzeichnet durch**
     Ausgeben einer Information, insbesondere einer akustischen oder optischen Warnmeldung an den Fahrer des Fahrzeugs, wenn das vorzugsweise gewichtete Ergebnis einen vorbestimmten Schwellenwert überschreitet.

13.  Computerprogramm (122) mit Programmcode für ein Steuergerät zum Erkennen einer Unaufmerksamkeit eines Fahrers eines Fahrzeugs,
     **dadurch gekennzeichnet,**
     **dass** der Programmcode ausgebildet ist zur Durchführung des Verfahrens nach einem der Ansprüche 1 - 12.

14.  Datenträger
     **gekennzeichnet durch**
     das Computerprogramm nach Anspruch 13.

## Claims

1.  Method for detecting when the driver of a vehicle, in particular a motor vehicle, becomes inattentive, comprising the following steps:

    - the detection of a movement of a steering wheel of a vehicle in the form of a steering wheel angle x (step S1); and
    - the detection of a steering inactivity phase and the determination of the magnitude of the degree of the steering inactivity phase by evaluating the detected steering wheel angle and/or its variation with time, wherein the degree of the steering inactivity phase is the period in which the steering wheel angle remains within a predetermined steering wheel angle interval ($\Delta$x) (step S2/2), the steering wheel angle interval being predetermined by the current speed of the vehicle (step S2/2); and

- the detection of a steering action following the steering inactivity phase and the determination of the magnitude of the degree of the steering action by evaluating the variation with time of the steering wheel angle (step 3/2); and

- the detection of a state of inattention if both the steering inactivity phase and the subsequent steering action are present to a predetermined minimum degree; and

- the determination of a measure for the severity of the inattention by evaluating the result of a connection of the degree of the steering inactivity phase and the degree of the steering action, wherein the connection of the degree of the steering inactivity phase and the degree of the steering action is on a characteristic map, on a weighting function or on a logical decision function.

2. Method according to claim 1,
   **characterised in that**
   the degree of the steering action following a steering inactivity phase is determined in the form of the then occurring maximum gradient of the steering wheel angle (step S3/3).

3. Method according to claim 2
   **characterised in that**
   the predetermined minimum degree of steering inactivity phase and steering action is present if the degree of the steering inactivity phase in the form of its duration exceeds a predetermined minimum duration and if the maximum gradient of the steering wheel angle exceeds a predetermined gradient threshold value (step S4/2).

4. Method according to any of claims 1 to 3,
   **characterised in that**
   the connection of the degree of the steering inactivity phase and the degree of the steering action is dimensioned in accordance with the speed of the vehicle and/or with the dynamics of the driving style of the driver of the vehicle.

5. Method according to any of claims 1 to 4,
   **characterised in that**
   in a subsequent step (step S3) the result of the connection is, in particular by means of the sigmoid function, mapped to a probability value P ($U_1$) between 0% and 100%, which represents the inattention of the driver at the time $t_1$.

6. Method according to claim 5,
   **characterised by**
   the following further steps (step S6) for assessing the fatigue of the driver:

   - the determination of a first probability vector $O_{n=1}$, the elements $O_{n=1, k1}$ of which represent probability values P ($O_{1, k1}$) for the occurrence of a probability value P ($U_1$) in individual predetermined and selected degree stages $k_1$ with $k_1 \, \varepsilon \, (1 \ldots K_1)$ (step S4); and
   - the determination of a fatigue probability vector S', the elements of which represent probabilities P (fatigue stage) for assigning the detected inattention of the driver when steering the vehicle to predetermined and suitably selected fatigue stages an accordance with the following formula (5):

$$S' (t) = O^T_1 \bullet B_1; \qquad\qquad (5)$$

   wherein
   $O^T_1$ is the transpose of the first probability vector;
   $B_1$ is the matrix B for predetermined conditional probabilities with reference to steering inattention, represented by the indicator n = 1 ; and
   $K_1$ is the number of degree stages for the indicator n = 1.

7. Method according to claim 6,
   **characterised by**

   - the determination of further probability vectors $O_{n=2} \ldots O_{n=N}$, the elements $O_{n, kn}$ with $k_n = 1 \ldots K_n$ represent probabilities P ($O_{n, kn}$) for the occurrence of probability values P ($U_n$) for other inattention indicators n = 2 ... N of the driver in addition to the steering inattention n = 1, in particular for the closing of the eye lids n = 2 or the reaction time n = 3, in degree stages $k_n$ individually predetermined for each inattention indicator, and
- the fatigue probability vector S' (step S6) is then calculated in accordance with the following formula (6):

$$S''(1) = \prod_{n=1}^{N} O^T{}_n \bullet B_n \qquad (6)$$

wherein

n is the nth indicator for the inattention of the driver;
$O^T{}_n$ is the transpose of the further probability vectors;
$B_n$ is the matrix B for the indicator n; and
N is the number of indicators.

**8.** Method according to claim 6 or 7,
**characterised by**

- the storage of the fatigue probability vector S''' (t-1); and
- the calculation of a further precised fatigue probability vector S''' (t) in accordance with the following formula (7) (step S7):

$$S''' (t) = S'' (t) \bullet A \bullet S''' (t-1), \qquad (7)$$

wherein
A is the matrix of the conditional probabilities between a fatigue stage from the last step and a current fatigue stage.

**9.** Method according to any of claims 1 to 8,
**characterised in that,**
in addition to steering inattention and the optional further indicators for the inattention of the driver, it is detected whether the driver is holding a conversation or operates a control such as the radio or the glove compartment of the vehicle; and **in that**, by evaluating these detected events using a probabilistic model, a statement can be made on the probability with which it can be assumed that either the distraction of the driver by the conversation or the operation of the control or else the fatigue of the driver is the cause for the observed inattention.

**10.** Method according to any of claims 1 to 5,
**characterised by** the following steps:

- the execution of the connection at various times ti with i = 1 -I during a predetermined measuring time interval;
- the storage of the results of the connections at the times ti together with the assigned weighting factors representing the driving situation or the current distraction of the driver at the times ti; and
- the calculation of a weighted result of the connections by mathematical, preferably arithmetical, averaging of the results stored during the measuring time interval, taking account of their assigned weighting factors.

**11.** Method according to claim 10,
**characterized in that**
the weighting factors are calculated taking account of circadian influencing factors and/or the time elapsed since the start of travel.

**12.** Method according to claim 10 or 11,
**characterised by**
the output of information, in particular an audible or visual alarm, to the driver of the vehicle if the preferably weighted result exceeds a predetermines threshold value.

**13.** Computer programme (122) for a control unit for the detection of an inattention of a driver of a vehicle,
**characterised in that**
the programme code is resigned for the execution of the method according to any of claims 1 to 12.

**14.** Data storage medium,
**characterised by**
the computer programme according to claim 13.


**Revendications**

**1.** Méthode pour reconnaître le manque d'attention du conducteur d'un véhicule, en particulier un véhicule à moteur, comprenant les étapes suivantes :

- détection d'un mouvement d'un volant de direction du véhicule sous forme d'un angle du volant de direction x (étape S1) ; et
- reconnaissance d'une phase d'inactivité de braquage et détermination de l'ampleur de l'importance de la phase d'inactivité de braquage par l'estimation de l'angle du volant de direction détecté et / ou par sa variation dans le temps, l'importance de la phase d'inactivité de braquage étant déterminée en tant que durée pendant laquelle l'angle du volant de direction reste dans un intervalle d'angle du volant de direction ($\Delta$x) (étape S2/2) prédéterminé, l'intervalle d'angle du volant de direction étant prédéterminé par la vitesse actuelle du véhicule (étape S2/2) ; et
- reconnaissance d'une action de braquage qui suit une phase d'inactivité de braquage et détermination de l'ampleur de l'importance de l'action de braquage par l'évaluation de la variation dans le temps de l'angle du volant de direction (étape S3/2) ; et
- reconnaissance d'un état du manque d'attention, lorsque tant la phase d'inactivité de braquage que l'action de braquage qui suit se situent dans une importance minimum prédéterminée ; et
- détermination d'un degré de gravité du manque d'attention en estimant le résultat d'une combinaison de l'importance de la phase d'inactivité de braquage et de l'importance de l'action de braquage, la combinaison, l'importance, la phase d'inactivité de braquage et l'importance de l'action de braquage s'établissent par un diagramme caractéristique, une fonction de pondération ou une fonction de décision logique.

**2.** Méthode selon la revendication 1
**caractérisée en ce que**
l'importance de l'action de braquage à la suite d'une phase d'inactivité de braquage précédente sera établie sous forme de gradients maximaux alors existants de l'angle du volant de direction (étape S3/3).

**3.** Méthode selon la revendication 2
**caractérisée en ce que**
l'importance minimale prédéterminée de la phase d'inactivité de braquage et de l'action de braquage existe lorsque l'importance de la phase d'inactivité de braquage sous forme de durée est plus grande qu'une durée minimale prédéterminée et que le gradient maximal de l'angle du volant de direction est supérieur à une valeur seuil de gradient prédéterminée (étape S4/2).

**4.** Méthode selon l'une quelconque des revendications 1 à 3,
**caractérisée en ce que**
la combinaison de l'importance de la phase d'inactivité de braquage et de l'importance de l'action de braquage est conçue en fonction de la vitesse du véhicule et / ou d'une dynamique de la conduite du conducteur du véhicule.

**5.** Méthode selon l'une quelconque des revendications 1 à 4,
**caractérisée en ce que**
dans une étape suivante (étape S3) le résultat de la combinaison est représenté sur une valeur de probabilité P$(U_1)$ comprise entre 0 et 100 %, en particulier au moyen de la fonction sigmoïde, laquelle valeur représente le manque d'attention du conducteur lors de la conduite du véhicule au moment $t_1$.

**6.** Méthode selon la revendication 5,
**caractérisée par**
les autres étapes suivantes (étape S6) pour évaluer la fatigue du conducteur :

- déterminer un premier vecteur de probabilité $O_{n=1}$, dont les éléments $O_{n=1, k1}$ à chaque fois des valeurs de probabilité P$(O_{1,k1})$ représentent qu'une valeur de probabilité P$(U_1)$ apparaît dans des niveaux d'importance individuels sélectionnés et prédéfinis $k_1$ avec $k_1 \in \{1...K_1\}$ (étape S4) ; et

- déterminer un vecteur de probabilité de fatigue S', dont les éléments à chaque fois des probabilités P (niveau de fatigue) représentent pour cela que le manque d'attention du conducteur détecté lors de la conduite du véhicule est affecté à des niveaux de fatigue individuels et sélectionnées de manière appropriée, conformément à la formule suivante :

$$S'(t) = O^T_1 \cdot B_1 ; \qquad\qquad (5),$$

sachant que

$O^T_1$ représente la transposée du premier vecteur de probabilité ;
B1 représente la matrice B des probabilités prédéfinies en fonction du manque d'attention au volant, représentée par l'indicateur n = 1 ; et
K1 représente le nombre des niveaux d'importance pour l'indicateur n = 1

**7.** Méthode selon la revendication 6,
**caractérisée par** les autres étapes suivantes :

- déterminer d'autres vecteurs de probabilité $O_{n=2}$... $O_{n=N}$, dont les éléments $O_{n, kn}$ avec $k_n = 1...K_n$ chaque fois des probabilités $P(O_{n, kn})$ représentent pour cela que les valeurs de probabilité $P(U_n)$ pour d'autres indicateurs de manque d'attention n = 2. N du conducteur, outre le manque d'attention au volant n = 1, en particulier le comportement de la fermeture des paupières n = 2 ou le temps de réaction n = 3, apparaissent dans chaque niveau d'importance $k_n$ prédéfini individuellement pour les indicateurs de manque d'attention, et
- calculer ensuite le vecteur de probabilité de fatigue S'' (étape S6) d'après la formule (6) suivante :

$$S'' = \prod_{n=1}^{N} O^T_n \cdot B_n \qquad\qquad (6)$$

sachant que

n représente l'indicateur n-$^{\text{éme}}$ pour le manque d'attention du conducteur ;
$O^T_n$ représente la transposée des autres vecteurs de probabilité ;
$B_n$ représente la matrice B pour l'indicateur n ; et
N représente le nombre d'indicateurs

**8.** Méthode selon l'une quelconque des revendications 6 ou 7,
**caractérisée par**

- la mémorisation des vecteurs de probabilité de fatigue S'''(t-1); et
- le calcul d'un autre vecteur de probabilité de fatigue S''' (t) encore plus précis selon la formule suivante (7) (étape 7) :

$$S'''(t) = S''(t) \cdot A \cdot S'''(t-1), \qquad\qquad (7)$$

sachant que

A représente la matrice des probabilités prédéfinies entre un niveau de fatigue provenant du dernier intervalle et un niveau de fatigue actuel.

**9.** Méthode selon l'une quelconque des revendications 1 à 8,
**caractérisée en ce qu'**
outre le manque d'attention au volant et les autres indicateurs optionnels pour le manque d'attention du conducteur, on peut également déterminer si le conducteur tient une conversation ou actionne un élément de réglage, par exemple met en marche la radio ou ouvre la boîte à gant du véhicule ; et que grâce à l'évaluation de ces événements détecté, à l'aide d'un modèle de probabilité il est possible de supposer avec quelle probabilité une distraction du

EP 1 548 678 B1

conducteur est due à la conversation ou à l'actionnement d'un élément de réglage et avec quelle probabilité la fatigue du conducteur peut supposée être la cause du manque d'attention observé.

10. Méthode selon l'une quelconque revendication 1 à 5,
**caractérisée par** les étapes suivantes :

- effectuer la combinaison de différents moments fixés ti avec i = 1-I pendant une période de mesure prédéterminée :
- mémoriser les résultats des combinaisons aux moments fixés ti à chaque fois avec des facteurs de pondération attribués, lesquels représentent la situation de conduite du véhicule ou la distraction actuelle du conducteur au moment fixé ti ; et
- calculer un résultat pondéré de la combinaison par le calcul d'une valeur moyenne mathématique, de préférence arithmétique des résultats mémorisés pendant la période de mesure en tenant compte de leurs facteurs de pondération attribués.

11. Méthode selon la revendication 10,
**caractérisée en ce que**
les facteurs de pondération sont calculés en tenant compte des facteurs d'influence circadiens et/ ou du moment du début de la conduite.

12. Méthode selon la revendication 10 ou 11,
**caractérisée par**
la sortie d'une information, en particulier un avertissement sonore ou optique au conducteur du véhicule lorsque le résultat de préférence pondéré dépasse une valeur seuil prédéterminée.

13. Logiciel (122) avec un code de programme pour un appareil de commande permettant de déceler un manque d'attention d'un conducteur d'un véhicule,
**caractérisé en ce que**
le code de programme est conçu pour mettre en oeuvre la méthode selon l'une quelconque des revendications 1 à 12.

14. Support de données,
**caractérisé par**
le logiciel selon la revendication 13.

Fig. 1

*Fig. 2*

Fig. 3a

Fig. 3b

Fig. 3c

*Fig. 4*

*Fig. 5*

*Fig. 6*

*Fig. 7*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19818239 A1
- US 6061610 A
- DE 2546345
- JP 07093678 A
- EP 0147539 A2
- US 5745031 A